Europäisches Patentamt

(19) European Patent Office

Office européen des brevets

(11) Publication number: **0 236 280 B1**

(12) **EUROPEAN PATENT SPECIFICATION**

(45) Date of publication of patent specification:
**12.06.91 Bulletin 91/24**

(51) Int. Cl.⁵: **A61K 47/10, A61K 31/57**

(21) Application number: **87830041.7**

(22) Date of filing: **02.02.87**

(54) **Injectable pharmaceutical formulations of active principles having general anaesthetic activity.**

The file contains technical information submitted after the application was filed and not included in this specification

(30) Priority: **04.02.86 IT 1928886**

(43) Date of publication of application:
**09.09.87 Bulletin 87/37**

(45) Publication of the grant of the patent:
**12.06.91 Bulletin 91/24**

(84) Designated Contracting States:
**AT BE CH DE ES FR GB GR IT LI LU NL SE**

(56) References cited:
**DE-A- 2 330 733**

(56) References cited:
**CHEMICAL ABSTRACTS, vol. 92, no. 1, 7th January 1980, page 113, abstract no. 114q, Columbus, Ohio, US; J.B. GLEN et al.: "An animal model for the investigation of adverse responses to i.v. anesthetic agents and their solvents", & BR. J. ANAESTH. 1979, 51(9), 819-27**

(73) Proprietor: **Mazzarella, Basilio**
**Via S. Pasquale a Chiaia 62**
**I-80121 NAPOLI (IT)**
Proprietor: **Mastronardi, Pasquale**
**Via Santo Stefano 23**
**I-80127 NAPOLI (IT)**

(72) Inventor: **Mazzarella, Basilio**
**Via S. Pasquale a Chiaia 62**
**I-80121 NAPOLI (IT)**
Inventor: **Mastronardi, Pasquale**
**Via Santo Stefano 23**
**I-80127 NAPOLI (IT)**

(74) Representative: **Bianchetti, Giuseppe**
**Studio Consulenza Brevettuale Via Rossini, 8**
**I-20122 Milan (IT)**

## Description

The present invention relates to injectable pharmaceutical formulations of poorly water-soluble active ingredients having an anaesthetic activity, particularly Alfaxalone, Alfadolone acetate and propanidid.

U.K. Patent no. 1 317 184 disclose Alfaxalone and Alfadolone acetate, well-known steroids having general anaesthetic activity, the combination thereof has been already used in human therapy. Propanidid is another known anaesthetic drug used in therapy, and it is described in German patent no. 1 134 981.

A common drawback to these compounds resides in their poor water-solubility, which opposes the formulation thereof in suitable solutions for the parenteral administration, which is basic for this kinds of drugs which are required to have a particularly fast action.

Therefore, formulations of said active ingredients based on tensides, particularly the polyoxyethylated castor oil known as CREMOPHOR EL (trade mark) have been proposed. However, the use of such a solvent has been prohibited by Health Authorities on international scale, several cases of anaphylactoid reactions, some of them with lethal results, having been evidenced, subsequently to administration of said anaesthetic formulations. The surfactant properties of Cremophor were hypotized to increase in some way immonogenicity of the active ingredients (British J. of Anaesthesia 48, 881-6, 1976a).

Due to the above problem, said important drugs, which are considered ideal intravenous anaesthetics, now are no more available from the Therapeutic Repertory, for lack of appropriate, safe, non-toxic and stable formulations.

Now it has been found, and it is the object of the present invention, that Alfaxolone, Alfadolone acetate and Propanidid may be conveniently formulated using a solvent mixture consisting of propylene glycol, ethanol and water in appropriate ratios.

The resulting solutions overcome the problems of the prior art and are perfectly tolerated. Moreover, they can be stocked for a long time, due to their particularly high stability.

The compositions of the present invention consist of percentages which may vary from 15 to 70%, of a mixture of propylene glycol and ethanol, percentages from 30 to 50% being particularly preferred. Deionized apyrogenic water, conventionally used for injections, completes the compositions. Propylene glycol-ethanol volume ratio is from 1 : 2 to 2 : 1, preferably 1,5 : 1.

Where Alfaxalone and Alfadolone acetate are the active ingredients, the concentration thereof will appropriately range between 1 and 10 mg/ml of active ingredient, using an Alfadolone/Alfaxalone 1 : 3 ratio, which is known in therapy. A preferred concentration range is from 3 to 7 mg/ml.

Moreover, Alfadolone acetate in the formulations of the invention may be replaced by the equivalent amount of Alfaxalone. Alfadolone is in fact known to be the less active of the two components, and it is generally used as an auxiliary solubilizer for the less soluble Alfaxalone.

As far as Propanidid is concerned, appropriate concentration of this active ingredient range from 10 to 100 mg/ml, preferably about 50 mg/ml. It is obvious that the compositions of the present invention may optionally contain buffering agent, sodium chloride or other excipients commonly used in pharmaceutical technique, provided, of course, they do not include Cremophor® or other tensides.

The following examples illustrate the invention in more detail.

## EXAMPLE 1

450 mg of 30α-hydroxy-5α-pregnan-11,20-dione (Alfaxolone) and 150 mg of 21-acetoxy-3α-hydroxy-5α-pregnan-11,20-dione (Alfadolone acetate) were dissolved in a mixture containing 20 ml of absolute ethanol and 30 ml of propylene glycol ; 50 ml of deionized water were slowly added to the solution, under continuous stirring. PH was adjusted to 5.60. After filtration, the solution was divided into 5 or 10 ml vials and sterilized under vapour stream for 30 minutes.

Each ml of the final solution contains :

| | |
|---|---|
| Alfaxalone | 4.5 mg |
| Alfadolone acetate | 1.5 mg |
| Absolute ethanol | 0.2 ml |
| Propylene glycol | 0.3 ml |
| Deionized water | 0.5 ml |

## EXAMPLE 2

The procedure of Example 1 was repeated, but using Propanidid instead of Alfaxalone and Alfadolone acetate, in appropriate amounts, to obtain an extremely stable injectable solution, having the following composition (referred to a 1 ml volume) :

| Propanidid | 50 mg |
|---|---|
| Absolute ethanol | 0.2 ml |
| Propyleneglycol | 0.3 ml |
| Deionized water | 0.5 ml |

## Claims

### Claims for the Contracting States : BE, CH, DE, FR, GB, IT, LI, LU, NL, SE, AT, ES, GR

1. Aqueous anaesthetic pharmaceutical compositions containig as the active ingredient Alfaxalone, Alfadolone acetate or Propanidid, characterized in containing propylene glycol and ethanol, in ratios from 1 : 2 to 2 : 1 by volume.

2. Pharmaceutical compositions according to claim 1, wherein the ethanol-propylene glycol ratio is 1 : 1.5, and the percentage thereof based on total solution volume is 50%.

## Ansprüche

### Ansprüche für die Vertragsstaaten : BE, CH, DE, FR, GB, IT, LI, LU, NL, SE, AT, ES, GR

1. Wäßriges, anaesthetisches pharmazeutisches Präparat, das als aktiven Bestandteil Alfaxalon, Alfadolonacetat oder Propanidid enthält, dadurch **gekennzeichnet**, daß es Propylenglycol und Ethanol in Verhältnissen von 1 : 2 bis 2 : 1, ausgedrückt durch das Volumen, enthält.

2. Pharmazeutisches Präparat nach Anspruch 1, dadurch **gekennzeichnet**, daß das Ethanol-Propylenglycol-Verhältnis 1 : 1,5 beträgt und daß der Prozentgehalt davon, bezogen auf das Gesamtlösungsvolumen, 50% beträgt.

## Revendications

### Revendications pour les états contractants : BE, CH, DE, FR, GB, IT, LI, LU, NL, SE, AT, ES, GR

1. Compositions pharmaceutiques anesthésiques aqueuses contenant à titre d'ingrédient actif de l'Alfaxalone, de l'Alfadolone acétate ou du Propanidide, caractérisées en ce qu'elles contiennent du propylène glycol et de l'éthanol dans des rapports en volume de 1 : 2 à 2 : 1.

2. Compositions pharmaceutiques selon la revendication 1, dans lesquelles le rapport éthanol-propylène glycol est de 1 : 1,5 et leur pourcentage basé sur le volume total de solution est de 50%.